Europäisches Patentamt

European Patent Office

Office européen des brevets

(11) Veröffentlichungsnummer: **0 311 555**
**A2**

## (12) EUROPÄISCHE PATENTANMELDUNG

(21) Anmeldenummer: 88730225.5

(22) Anmeldetag: 05.10.88

(51) Int. Cl.⁴: **A 61 B 17/58**

(30) Priorität: 06.10.87 DE 3734108

(43) Veröffentlichungstag der Anmeldung:
12.04.89 Patentblatt 89/15

(84) Benannte Vertragsstaaten:
AT BE CH DE ES FR GB GR IT LI NL SE

(71) Anmelder: **MECRON MEDIZINISCHE PRODUKTE GMBH**
**Nunsdorfer Ring 25-27**
**D-1000 Berlin 48 (DE)**

(72) Erfinder: **Sievers, Uve Dr.**
**Ludwig-Guttmann-Strasse 13**
**D-6700 Ludwigshafen (DE)**

(74) Vertreter: **Christiansen, Henning, Dipl.-Ing.**
**Patentanwalt CHRISTIANSEN Pacelliallee 43/45**
**D-1000 Berlin 33 (DE)**

(54) **Marknagel für die Behandlung von Knochenbrüchen.**

(57) Marknagel (1) für die Behandlung von Knochenbrüchen nach dem Prinzip der Markraumnagelung, bestehend aus einem röhrenförmigen, kohlenstoffaserverstärkten Kunststoff, der in Längsrichtung des Marknagels (1) leicht gebogen ist und eine zum Ende sich konisch verjüngende und von außen scharf angeschliffene Spitze (2) aufweist.

Fig.1

EP 0 311 555 A2

Beschreibung

## Marknagel für die Behandlung von Knochenbrüchen

Die Erfindung bezieht sich auf einen Marknagel für die Behandlung von Knochenbrüchen nach dem Prinzip der Markraumnagelung.

Beispielsweise aus der Literaturstelle "Die Bündel-Nagelung", (Springer-Verlag, Berlin, Göttingen, Heidelberg 1961, Seiten 3 bis 26 und 56 bis 58) ist es bekannt, zur Behandlung von Brüchen langer Röhrenknochen die Methode der Marknagelung anzuwenden, bei der ein metallischer Marknagel über die Bruchstelle durch die Markhöhle des betreffenden Knochens getrieben und in der Markhöhle zur Stabilisierung der Fraktur nach entsprechender Reposition des Knochens verankert wird. Nach dem vollständigen Ausheilen des Bruches wird dann der Marknagel wieder entfernt. Die angestrebte Stabilisierung des Bruches bei der Marknagelung wird durch Quer- oder Längsverklemmung des Marknagels erzielt, der unterschiedliche Querschnittsformen aufweisen kann.

Die bekannten metallischen Marknägel weisen jedoch eine Reihe von Nachteilen auf, wobei bei den starren metallischen Nägeln die Gefahr der Knochenspaltung, des Verfangens der Nagelspitze und der Nagelverkrümmung sowie der Achsenknickung und der mangelnden Biegungsstabilität sowie Drehstabilität gegeben ist. Andere Markraumnagelungen wie beispielsweise die Bündel-Nagelung mit einem Bündel dünner elastischer Stahlnägel sind hinsichtlich ihrer nicht immer gewährleisteten Stabilität und der Notwendigkeit eines Einhaltens einer bestimmten Behandlungstaktik nachteilig.

Zur Behandlung per- und subtrochanterer Brüche nach dem Prinzip der Markraumnagelung ist auch die Verwendung sogenannter Federnägel bekannt, die im Markrohr elastisch ververspannt angeordnet und an dem der Einschlagstelle gegenüberliegenden Ende fächerförmig verteilt sind. Bei einer Belastung des auf dieser Weise stabilisierten Knochens verteilt sich der Druck über die gesamte Länge der Nägel gleichmäßig auf den Knochen. Die bekannten Federnägel geben im proximalen Bruchstück einen guten Halt sowie eine gute Rotationsstabilität und ermöglichen infolge ihrer Federkraft ein Auffädeln der Fragmente und ein exaktes Repositionieren der Fragmente durch Drehen am Vorschlaginstrument. Wie bei der Verwendung starrer Marknägel müssen Federnägel aus einem hochwertigen metallischen Werkstoff hergestellt werden, um die erforderlichen Festigkeits- und Elastizitätseigenschaften zu erzielen und eine Beeinträchtigung des Knochengewebes zu vermeiden. Bei der Verwendung von Federnägeln sind darüber hinaus üblicherweise drei und mehr Nägel zur Fixierung pertrochanterer Brüche erforderlich.

Bei den bekannten Knochennägeln besteht der Nachteil, daß sie vom Werkstoff her Fremdkörper bilden, so daß die Extraktion durch eine zweite Operation in jedem Fall erforderlich ist. Weiterhin sind sie in ihren Eigenschaften denen des umgebenden Knochenmaterials nicht angepaßt, so daß bei Belastung des Knochens aufgrund des unterschiedlichen Belastungsverhaltens schädliche Relativbewegungen entstehen.

Der Erfindung liegt die Aufgabe zugrunde, einen Knochennagel aus einem Material anzugeben, welches im Normalfall im Körper verbleiben kann und sich im Falle von Reoperationen zur Einfügung anderer Endoprothesen wie Knochensubstanz verhält und mit den üblichen Werkzeugen bearbeitet werden kann.

Diese Aufgabe wird durch die im kennzeichnenden Teil des Anspruchs 1 angegebenen Merkmale gelöst.

Der Erfindung beruht auf der Erkenntnis, daß beim Ersatz von Stahlwerkstoffen für die Osteosynthese ein Material gefunden werden muß, welches einerseits eine hohe Belastbarkeit aufweist und zum anderen relativ leicht ist und sich ferner in seinem Verformungsverhalten unter Belastung dem benachbarten Knochenmaterial anpaßt, so daß der Verbund Knochen-/Synthesematerial eine dynamische Einheit darstellt, so daß ein an der Grenze zwischen Knochen-und Synthesematerial erzielter Verbund auch bei Belastung erhalten bleibt.

Ferner ist vorteilhaft, daß bei - insbesondere bei älteren oder Tumorpatienten notwendigen Nachoperationen die Endoprothese an Ort und Stelle verbleiben kann bzw. so nachgearbeitet werden kann, daß Prothesenverlängerungen oder andere Endoprothetikteile eingesetzt werden können, ohne daß Werkzeuge zur Metallbearbeitung eingesetzt werden müßten, was nicht ohne tiefgreifende Schädigung des benachbarten Gewebes möglich ist.

Günstig ist weiterhin, daß ein Verbund mit weiteren Endoprothetikteilen durch Nachbearbeitung, wie Erzeugen von Gewinden etc. mit einfachen Knochenbearbeitungsmaterialien - also entsprechend dem Anschluß an normale Knochensubstanz - erzielt werden kann. Dabei anfallende Späne stellen - im Gegensatz zu Metallspänen - keine Gefahr für den Organismus dar und es braucht nicht eine besondere Sorgfalt darauf verwandt zu werden, diese zu entfernen.

Vorteilhaft ist ferner, daß ein als Hohlkörper ausgebildeter Nagel nicht - wie bspw. ein Küntschernagel - ge schlitzt ausgeführt werden muß, um die zum Eintreiben erforderliche Elastizität zu erhalten. Durch die Ausbildung als geschlossener zylindrischer Hohlkörper ist einerseits die zum Eintreiben erforderliche Elastizität vorhanden - andererseits bleibt auch die Torsionssteifigkeit erhalten, welche beim Küntschernagel wegen der Möglichkeit der Längsverschiebung der beiden Kanten im Bereich des Schlitzes sehr stark herabgesetzt ist.

Es wird dabei ein Marknagel für die Behandlung von Knochenbrüchen nach dem Prinzip der Markraumnagelung geschaffen, der bei hoher Biegesteifigkeit und Rotationsstabilität sowie geringer Materialermüdung unter Belastung eine hohe Elastizität beim Eintreiben des Marknagels mit geringem Kraftaufwand sicherstellt, der einfach und billig herstellbar und in optimaler Weise Gewebeverträglich und in der Lage ist, die Fragmente allein unter Verwendung eines Marknagels sicher zu stabilisieren.

Die erfindungsgemäße Lösung stellt bei hoher Biegesteifigkeit und Rotationsstabilität sowie geringer Materialermüdung unter Belastung des Marknagels eine hohe Elastizität beim Eintreiben des Marknagels mit geringem Kraftaufwand sicher, der Marknagel ist einfach und günstig herstellbar und optimal gewebeverträglich und in der Lage, allein die Fragmente eines Bruches sicher zu stabilisieren.

In einer vorteilhaften Weiterbildung weist der Marknagel eine im Querschnitt hohlzylindrische Form und eine an der Spitze des Marknagels vorgesehene, scharf geschliffene Ringschneide auf.

In einer weiteren vorteilhaften Weiterbildung besteht der Marknagel aus einem aus Kohlenstoffasern gewirkten Schlauch und einem aushärtbaren Kunstharz. Der Schlauch kann im Kreuzverbund angeordnete Kohlenstoffasern aufweisen, während das aushärtbare Kunstharz aus einem bioverträglichen TEEC-Matrixmaterial bestehen kann, in das die Kohlenstoffasern eingebettet sind und das bei einer hohen Temperatur von vorzugsweise 380°C aushärtet.

In einer weiteren vorteilhaften Ausgestaltung ist der Marknagel C-förmig gebogen. Zusätzlich kann er ein in den Marknagelkopf eingesetztes, metallisches Krafteinleitungselement aufweisen, das beispielsweise aus einem Hohlzylinder bestehen kann, dessen Innenwand eine rauhe Oberfläche aufweist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 einen Längsschnitt durch einen Marknagel aus kohlenstoffaserverstärktem Kunststoff;

Figur 2 eine vergrößerte Darstellung der Spitze des Marknagels und

Figur 3 eine vergrößerte Darstellung des Marknagelkopfes.

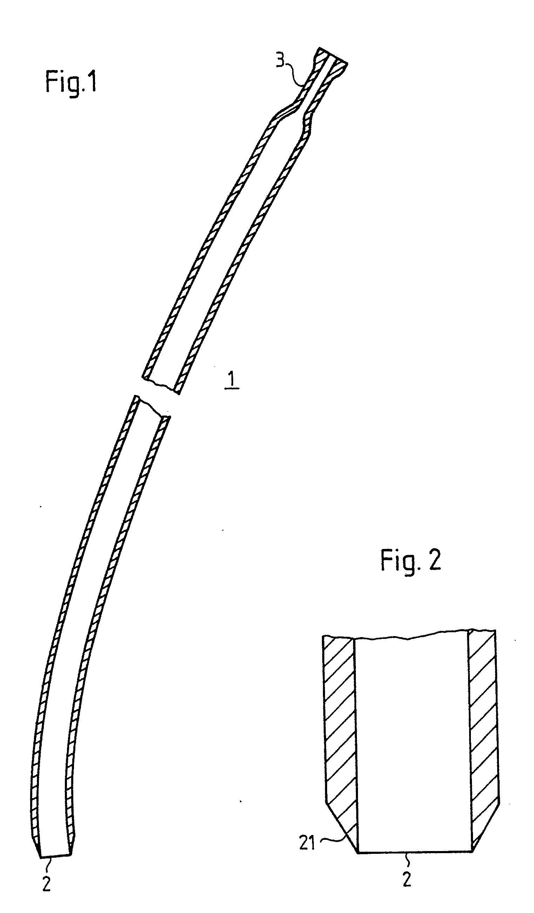

Der in Figur 1 dargestellte Längsschnitt durch einen röhrenförmigen Marknagel aus kohlenstoffaserverstärktem Kunststoff 1 ist in Längsrichtung C- bzw. J-förmig leicht gebogen und weist eine Spitze 2 sowie einen verengten Kopf 3 auf. Der Durchmesser des Marknagels beträgt im Bereich der Spitze ca. 10 Millimeter. Vorzugsweise ist der röhrenförmige Marknagel hohlzylindrisch ausgebildet.

Die Herstellung des Marknagels aus kohlenstoffaserverstärktem Kunststoff erfolgt unter Verwendung eines vorzugsweise im Kreuzverbund gewirkten Schlauches aus Kohlenstoffasern und eines aushärtbaren Kunststoffes, mit dem der gewirkte Schlauch getränkt wird. Das aushärtbare Kunstharz besteht vorteilhafterweise aus einem bioverträglichen TEEC-Matrixmaterial, in das die Kohlenstoffasern eingebettet sind und das bei einer hohen Temperatur von vorzugsweise 380°C aushärtet.

Alternativ können als aushärtbarer Kunststoff Werkstoffe, wie zum Beispiel Polyamid 6 oder Polyamid 6,6, sowie Kunststoffe Verwendung finden, oder solche, die üblicherweise als Massenkunststoffe bezeichnet werden, wie zum Beispiel Polyolefine (Polyäthylen, Polypropylen). In Verbindung mit den erfindungsgemäßen Kohlenstoffasern ist ein hochfester Markraumnagel gegeben, der sich durch hohe Zugfestigkeit und Steifigkeit sowie hohe Wärmestandfestigkeit auszeichnet, die normalerweise nur von metallischen Werkstoffen erzielt werden können bzw. diese Eigenschaften der metallischen Werkstoffe sogar übertreffen.

Die C- bzw. J-Form des Marknagels kann durch Einbringen eines geeigneten Kerns in Form beispielsweise eines zylindrischen Drahtes entsprechenden Außendurchmessers erfolgen, um den der gewirkte Schlauch aus Kohlenstoffasern gezogen wird. Nach dem Aushärten des aufgebrachten Kunstharzes kann der Kern entfernt werden und eine weitere Bearbeitung des Marknagels aus kohlenstoffaserverstärktem Kunststoff 1 erfolgen.

Um eine glatte Oberfläche des Marknagels zu erzielen, kann das Aufbringen des aushärtbaren Kunstharzes unter hohem Druck erfolgen oder ein anschließendes Polieren der Oberfläche vorgenommen werden.

Die Verwendung eines kohlenstoffaserverstärkten Kunststoffmaterials für die Herstellung eines Marknagels in Verbindung mit einem geeigneten Durchmesser des Marknagels gewährleistet, daß beim Eintreiben des Marknagels in die Markhöhle eines Knochens eine exakte Führung des Marknagels von der Eintreibstelle aus möglich ist, ohne daß der Marknagel abknickt oder unkontrollierte Bewegungen ausführt. Die scharf geschliffene Ringschneide an der Spitze des Marknagels gestattet ein Eintreiben des Marknagels mit geringem Kraftaufwand und vermeidet ein Zerquetschen von Knochenmark während des Eintreibvorgangs.

In eingesetzten Zustand des Marknagels zeichnet sich derartiges Marknagel durch seine hohe Rotationsstabilität und seine Fähigkeit aus, auch stärkste Schwingungen ohne Materialermüdung aufzunehmen. Ein weiterer Vorteil ist seine gute Gewebeverträglickeit und die Vermeidung jedweder Einflüsse auf das Knochenmark wie sie beispielsweise bei der Verwendung von Marknägeln aus Stahl durch die Korrosion gegeben ist. Die zylindrisch leicht gebogene Form in Ver bindung mit seinem Durchmesser ermöglicht eine sichere Anlage des Marknagels in der Markhöhle des Knochens und schafft somit die Voraussetzung dafür, daß die Fragmente unter Verwendung nur eines Marknagels stabilisierbar sind.

Die Extraktion des Marknagels nach ausgeheiltem Bruch kann in einfacher Weise durch Ergreifen des verengten oder abgeflachten Kopfes 3 des Marknagels erfolgen, ohne daß die Gefahr besteht, daß der Marknagel abreißt.

In Figur 2 ist in vergrößertem Maßstab die Spitze 2 des Marknagels 1 dargestellt und verdeutlicht die sich konisch leicht verjüngende Außenfläche 21, wobei durch scharfes Anschleifen der Spitze die Bildung einer Ringschneide geschaffen wird.

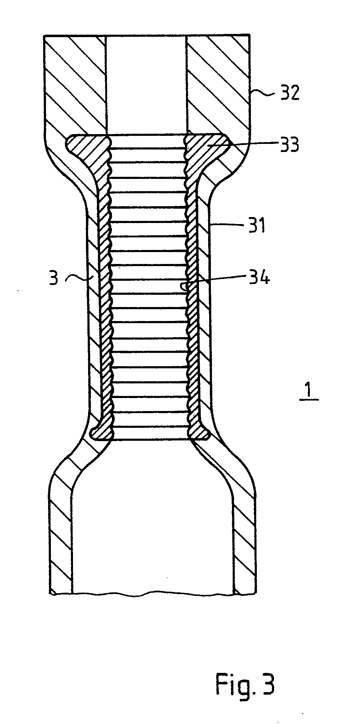

Figur 3 zeigt im vergrößerten Querschnitt den Marknagelkopf 3, der aus einem zylindrisch verengten oder abgeflachten Teil 31 und einem nach außen verdickten, umgebogenen oder gebördelten Endstück 32 besteht. In das zylindrisch verengte Teil 31 ist ein metallisches Krafteinleitungselement 34 in Form eines Hohlzylinders aus Titan eingesetzt, dessen Innenfläche rauh, vorzugsweise rillen-oder gewinderillenförmig ausgebildet ist, damit der Kopf 3 zur Extraktion des Marknagels 1 aus der Markhöhle bzw. zur Lageveränderung der Nagelspitze zur Reposition mit einem geeigneten Werkzeug, beispielsweise einer Zange ergriffen, reibschlüssig gehalten und herausgezogen werden kann.

Darüberhinaus kann der verengte oder abgeflachte Kopf 3 des hohlzylindrischen Marknagels 1 die Anbringung eines geeigneten Werkzeuges zum Eintreiben des Marknagels in die Markhöhle eines Knochens erleichtern.

Das metallische Krafteinleitungselement 34 kann bei der Herstellung des Marknagels I in das Gewebe aus Kohlenstoffasern eingesetzt und mit dem Kunststoff beim Aushärten verbunden werden, so daß eine feste Verbindung des eigentlichen Marknagelkopfes mit dem metallischen Krafteinleitungselement entsteht.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Marknagel für die Behandlung von Knochenbrüchen nach dem Prinzip der Markraumnagelung, **gekennzeichnet durch** einen röhrenförmigen, kohlenstoffaserverstärkten Kunststoffkörper, der in Längsrichtung des Marknagels (1) leicht gebogen ist und eine zum Ende sich konisch verjüngende und von außen scharf angeschliffene Spitze (2) aufweist.

2. Marknagel nach Anspruch 1, **gekennzeichnet durch** eine im Querschnitt hohlzylindrische Form und eine an der Spitze des Marknagels (1) vorgesehene, scharf geschliffene Ringschneide (21).

3. Marknagel nach Anspruch 1 oder 2, **dadurch gekennzeichnet,** daß die Kohlenstoffasern schlauchförmig gewirkt sind.

4. Marknagel nach Anspruch 3, **dadurch gekennzeichnet ,** daß der Schlauch im Kreuzverbund angeordnete Kohlenstoffasern aufweist.

5. Marknagel nach einem der vorangehenden Ansprüche, **dadurch gekenneichnet,** daß das aushärtbare Kunststoff aus einem bioverträglichen TEEC-Matrixmaterial besteht, in das die Kohlenstoffasern eingebettet sind und das bei einer hohen Temperatur von vorzugsweise 380°C aushärtet.

6. Marknagel nach einem der vorangehenden Ansprüche, **dadurch gekennzeichnet,** daß der Marknagel (1) J- oder C-förmig gebogen ist.

7. Marknagel nach einem der vorangehenden Ansprüche, **gekennzeichnet durch,** ein in den Marknagelkopf (3) eingebettetes, metallisches Krafteinleitungselement (33).

8. Marknagel nach Anspruch 7, **dadurch gekennzeichnet,** daß das metallische Krafteinleitungselement (33) aus einem Hohlzylinder besteht, dessen Innenwand eine rauhe Oberfläche aufweist.

9. Marknagel nach Anspruch 8, **dadurch gekennzeichnet,** daß die rauhe Oberfläche radial oder gewindeförmig verlaufende Rillen (34) aufweist.

10. Marknagel nach einem der vorangehenden Ansprüche 7 bis 9, **dadurch gekennzeichnet,** daß das metallische Krafteinleitungselement (33) aus Titan besteht.

11. Marknagel nach Anspruch 2, **dadurch gekennzeichnet,** daß der Marknagel (1) im Bereich seiner Spitze (2) einen Durchmesser von 10 Millimeter aufweist.

Fig.1

Fig. 2

Fig. 3